# EUROPÄISCHE PATENTSCHRIFT

(11) **EP 1 657 524 B1**
(45) Veröffentlichungstag und Bekanntmachung des Hinweises auf die Patenterteilung: **09.01.2008**
(21) Anmeldenummer: 05110667.2
(22) Anmeldetag: 11.11.2005
(51) Int. Cl.: G01B 11/24, A61C 13/00, H04N 1/387

(54) **Verfahren und Vorrichtung zur 3D-Vermessung von Zahnmodellen und Verschiebeplatte dazu**
Method and apparatus for the 3D measurement of dental models and a slidable plate for the same
Procédé et dispositif pour la mesure en 3D des modèles de dents et un plat coulissant pour le même

(30) Priorität: 12.11.2004 DE 102004054876
(43) Veröffentlichungstag der Anmeldung: 17.05.2006
(60) Teilanmeldung: 07121192.4
(73) Patentinhaber: Sirona Dental Systems GmbH, 64625 Bensheim (DE)
(72) Erfinder: Eiff, Wolfgang, 64646 Heppenheim (DE); Fornoff, Peter, 64385 Reichelsheim (DE)
(74) Vertreter: Sommer, Peter

(56) Entgegenhaltungen:
- WO-A-03/062740
- DE-A1- 4 301 538
- DE-C1- 19 924 291
- GB-A- 2 397 896
- US-A1- 2003 068 079
- US-A1- 2004 201 856
- PATENT ABSTRACTS OF JAPAN Bd. 1997, Nr. 11, 28. November 1997 (1997-11-28) & JP 09 178437 A (YUNISUN:KK), 11. Juli 1997 (1997-07-11)

## Beschreibung

Die Erfindung betrifft eine Vermessungseinrichtung zur dreidimensionalen Vermessung von Zahnmodellen sowie ein Verfahren dazu.

### Stand der Technik

Aus dem Stand der Technik ist es bekannt, Zahnabdrücke oder aus Zahnabdrücken gewonnene Zahnmodelle extraoral mit stationären Vermessungseinrichtungen zu vermessen. Meist ist das zu vermessende Objekt größer als der Aufnahmebereich der Bildaufnahmeeinheit. Daher ist es notwendig, das aufzunehmende Objekt in mehreren Teilen aufzunehmen und die relative Position der Bildaufnahmeeinheit zu dem zu vermessenden Objekt für jede Aufnahme zu variieren.

In der EP 0 913 130 A1 wird eine Vorrichtung zum Ermitteln der Form eines Duplikats offenbart, die eine Einspannvorrichtung, eine Sensoreinrichtung und eine Speichereinheit enthält, bei der die Einspannvorrichtung und die Sensoreinrichtung relativ zueinander bewegbar sind.

Die einzelnen Aufnahmen werden durch Softwarekorrelation miteinander verbunden. Die Software versucht dabei, in zwei verschiedenen Aufnahmen gleiche markante Punkte zu finden und damit eine Lagebeziehung zwischen den Aufnahmen festzustellen. Aufgrund der Abbildungseigenschaften und weiterer softwarebedingter Ungenauigkeiten bei der Softwarekorrelation gelingt die Zusammensetzung zweier korrelierter Bilder in der Praxis nicht ohne Fehler. Bei der Aneinanderreihung mehrerer Aufnahmen können sich diese Fehler im ungünstigsten Fall addieren.

Darüber hinaus ist der rechentechnische Aufwand für die Korrelation von Bildern mit unbekannter Verschiebung sehr hoch, so dass die Verarbeitungszeiten lang sind.

Aus der US 2003/0068079 A1 ist eine 3D-Vermessungseinrichtung für die Computergestützte Zahnrestauration bekannt, wobei für das berührungslose Abtasten des Zahnmodells ein Drehtisch vorgesehen, der für eine lineare Verstellbewegung auf einem linear beweglichen Maschinenbett gelagert ist. Das Maschinenbett verfügt dazu über Führungsfortsätze, die auf einer Schiene gleiten. Der Antrieb erfolgt über einen Schrittmotor, so dass abhängig von der Schrittzahl eine Positionsveränderung von der Aufnahme zur nächsten durchgeführt werden kann.

Aus der DE 4301538 A1 ist ein Verfahren und eine Anordnung zur berührungslosen dreidimensionalen Messung von Gebissmodellen bekannt. Das Messgut ist auf einer rotatorischen Verstelleinheit angeordnet und der Messkopf wird durch eine dreiachsige lineare Verschiebeeinrichtung geführt. Mit zwei weiteren Drehachsen wird die erforderliche freie Positionierbarkeit des Messkopfs erreicht. Einzelne Datensätze können nach einer Reduktion zu einem Gesamtdatensatz zusammengefasst werden, der das zu vermessende Objekt umfassend beschreibt.

Aus der GB 2397896 A ist eine Montagevorrichtung beschrieben, bei der ein Grundplatte Vertiefungen aufweist und eine darauf anzuordnende Platte Vorsprünge. Die Vertiefungen können V-Nuten und die Vorsprünge können kugelförmig sein. Die Verschiebung von einer ersten Position in eine um einen vorgegebenen Abstand verschiedene zweite Position erfolgt durch Umsetzten des Verschiebeteils, etwa um Testdaten zu erhalten.

Es stellt sich somit die Aufgabe, eine Vermessungseinrichtung sowie ein Verfahren zur Vermessung von Zahnmodellen anzugeben, die eine schnelle und exakte Vermessung von Zahnmodellen zulässt und darüber hinaus gegenüber heutigen Geräten preiswert herzustellen ist.

### Offenbarung der Erfindung

Diese Aufgabe wird gelöst durch eine Vermessungseinrichtung gemäß dem Anspruch 1 sowie durch ein Verfahren zur Vermessung von Zahnmodellen gemäß dem Anspruch 12. Vorteilhafte Weiterbildungen sind in den Unteransprüchen beschrieben.

Die erfindungsgemäße Vermessungseinrichtung zur 3D-Vermessung von Zahnmodellen weist eine Aufnahmevorrichtung und eine Positionierungseinrichtung auf, wobei die Positionierungseinrichtung eine bezüglich der Aufnahmeeinrichtung positionierbare Platte enthält, auf der das zu vermessende Objekt befestigt werden kann. Die positionierbare Platte wird auf einer bezüglich der Aufnahmevorrichtung feststehenden Grundplatte verschoben. Die Platte ist als Verschiebeplatte ausgebildet und enthält erste Rastmittel. Die Grundplatte ist als Rastplatte ausgebildet und enthält zweite Rastmittel, die mit den ersten Rastmitteln so zusammenwirken, dass die Verschiebeplatte eine von mehreren vorgegeben Positionen zur Rastplatte einnehmen kann und in der ausgewählten Position einrastet. Die Aufnahmevorrichtung ist als Bilderfassungseinheit gestaltet. Damit ist es möglich, eine dreidimensionale Vermessung ohne den Einsatz von mechanischen Tastmitteln durchzuführen. Die Abstände der Rastpositionen in X- und in Y-Richtung sind so gewählt, dass diese kleiner oder gleich dem Bilderfassungsbereich der Bilderfassungseinrichtung und größer als die halbe Erstreckung des Bilderfassungsbereichs in die jeweilige Richtung sind. Der Abstand sollte so gewählt sein, dass ein nahtloses Aneinanderfügen oder eine Überlappung von Aufnahmen von benachbarten Rastpositionen existiert, damit eine lückenlose Zusammensetzung der Aufnahmen sichergestellt ist und der Überlappungsbereich klein genug ist, um eine effiziente Vermessung des aufzunehmenden Bereichs mit möglichst wenigen Aufnahmen zu gewährleisten.

Mit einer derart gestalteten Vermessungseinrichtung kann die positionierbare Platte nur exakt bestimmte Positionen einnehmen. Es existiert somit eine endliche Anzahl von Verschiebungsvektoren zwischen einzelnen Aufnahmen, die die Korrelation der verschiedenen Aufnahmen auf eine diskrete Anzahl von Korrelationsmöglichkeiten reduziert. Der notwendige Rechenaufwand wird dadurch erheblich reduziert. Darüber hinaus pflanzen sich Korrelationsfehler nicht mehr additiv fort. Die Vermessung ist somit genauer als eine Vermessung nach dem Stand der Technik.

Besonders vorteilhaft ist es, wenn der Bilderfassungsbereich der Bilderfassungseinheit kleiner als das aufzunehmende Objekt ist. Solche Bilderfassungseinheiten sind besonders günstig herzustellen, da die Auflösung des Bildsensors und/oder die Größe der Optik klein gehalten werden kann, was eine preiswerte Konstruktion mit Standardkomponenten ermöglicht.

Eine weitere besonders vorteilhafte Ausgestaltung der Erfindung betrifft die Verwendung einer Mund-Messkamera zur Bilderfassung. Mund-Messkameras sind im Stand der Technik bekannt und aufgrund der Möglichkeit der Verwendung von Gleichteilen und des geringeren Entwicklungsaufwands kann eine Vermessungseinrichtung bei Verwendung eine Mund-Messkamera preiswerter verkauft werden als eine Vermessungseinrichtung mit einer speziell entwickelten Bilderfassungseinrichtung.

Vorteilhafterweise ist die Vermessungseinrichtung mit einem Lichtfilter ausgestattet, das das Lichtein- und Austrittsfenster gegen Umgebungslicht abschirmt. Umgebungslicht verschlechtert die Qualität der Aufnahmen und in der Folge die Qualität der Vermessungsdaten. Das Lichtfilter kann so angebracht sein, dass es das Objektiv der Vermessungseinrichtung zusätzlich vor mechanischer Beschädigung schützt, indem es dem Objektiv vorgelagert angebracht wird.

Vorteilhafterweise arbeitet die Vermessungseinrichtung, insbesondere eine Mund-Messkamera, mit einem telezentrischen Strahlengang. Ein telezentrischer Strahlengang bringt den Vorteil mit sich, dass die Objektabbildung unabhängig vom Abstand des Objektes zum Objektiv immer gleich groß ist. Die Auswertung eines telezentrisch aufgenommenen Bildes wird durch diese Eigenschaft erleichtert.

Vorteilhafterweise ist der Abstand der Bilderfassungseinheit zu der Verschiebeplatte verstellbar. Die Tiefenschärfe aller optischen Abbildungseinrichtungen ist naturgegeben begrenzt. Da in der Praxis nicht alle zu vermessenden Objekte die selbe Höhe haben, kann es nötig sein, die Bilderfassungseinheit so zu verschieben, dass das zu vermessende Objekt innerhalb des Tiefenschärfebereichs liegt.

Vorteilhafterweise sind Mittel zum Erfassen des Abstands der Bilderfassungseinheit von der Rastplatte vorhanden. Auch bei telezentrischen Strahlengängen kann es unter Umständen notwendig sein, den relativen Abstand von der Bilderfassungseinheit zu dem zu vermessenden Objekt zu kennen, beispielsweise um eine Korrelation aufeinanderfolgender Aufnahmen zu ermöglichen, die mit unterschiedlichen Kamerahöhen vorgenommen wurden.

Besonders vorteilhaft ist es, wenn die Abstände zwischen den Rastpositionen in x- und y-Richtung so gewählt sind, dass sich die in benachbarten Positionen aufgenommenen Bildbereiche um mindestens 1/10 des Bilderfassungsbereichs überlappen. Dann ist die Überlappung ausreichend, um gleiche Bildteile in benachbarten Aufnahmen zu identifizieren und eine Korrelation sicherzustellen.

Vorteilhafterweise sind die einen Rastmittel als Erhöhung und die anderen Rastmittel als Vertiefung ausgebildet. Derartige Rastmittel sind mit einfachen Fertigungstechniken herstellbar und weisen trotzdem eine hohe Präzision auf.

Vorteilhafterweise sind an der Verschiebeplatte oder an der Rastplatte drei Erhöhungen vorgesehen. Eine dreibeinige Auflage gewährleistet einen sicheren und wackelfreien Sitz der Verschiebeplatte.

Vorteilhafterweise haben die Erhöhungen im Wesentlichen konvex gestaltete Auflageflächen. Solche Erhöhungen sind unempfindlich gegen Verformungen und haben meist einen exakt definierbaren Auflagepunkt.

Vorteilhafterweise sind die Vertiefungen als Nuten ausgeführt. Dann lässt sich die Verschiebeplatte auf leichte Weise in eine Richtung verschieben, weil mindestens eine der Erhöhungen in einer der Nuten gleitet, während die Verschiebeplatte verschoben wird.

Vorteilhafterweise ist der Übergang zwischen zwei Nuten konvex gestaltet. Dies schließt eine Fehlpositionierung der Verschiebeplatte in der Praxis nahezu aus, da die Erhöhung durch die Schwerkraft bedingt automatisch in die Vertiefung rutscht.

Es erweist sich als besonders vorteilhaft, wenn die zweiten Rastmittel in einen ersten Bereich mit parallelen, gleich beabstandeten Nuten und in einen zweiten Bereich mit parallelen, gleich beabstandeten Nuten aufgeteilt sind, wobei die Nuten des ersten Bereichs im Wesentlichen senkrecht zu den Nuten des zweiten Bereichs stehen. Dann lässt sich die Verschiebeplatte nur in X- und in Y-Richtung verschieben. Die Herstellung einer solchen Rastmittelstruktur erweist sich darüber hinaus als besonders einfach.

Vorteilhafterweise ist die Verschiebeplatte mit einer Vorrichtung zur Verstellung der Neigung des zu vermessenden Objekts ausgestattet.

Teilweise ist es nötig, Objekte nicht nur aus einer vorgebenden Richtung zu vermessen, sondern das Objekt bezüglich der Bildaufnahmeeinheit in eine andere Lage zu bringen, da manche Objekte Hinterschneidungen aufweisen, die andernfalls nicht erfasst werden können.

Vorteilhafterweise sind auf der Verschiebeplatte Markierungen angebracht, deren Position der Position der ersten Rastmittel auf der Unterseite entspricht. Der Bediener der Vermessungseinrichtung kann somit auf einfache Weise die Position der ersten Rastmittel auf der Rastplatte überprüfen.

Besonders vorteilhaft ist es, wenn die Rastplatte mindestens eine Durchbrechung zur Befestigung eines zu vermessenden Objektes enthält, das in X- sowie in Y-Richtung verschiebungsfest vermessen wird, wobei die Ausdehnungen des zu vermessenden Objektes kleiner sind als der Bilderfassungsbereich der Bilderfassungseinheit. Die Befestigung des Objekts geschieht über Befestigungsmittel, wobei die Befestigungsmittel und die Durchbrechung so angeordnet sind, dass das Objekt im selben Bilderfassungsbereich wie das mittels der Verschiebeplatte verschiebbar zu vermessende Objekt liegt. Das Objekt kann dabei auch schräg angeordnet sein, wofür eine zweite Durchbrechung vorgesehen sein kann um sicherzustellen, dass das Objekt trotz Schrägstellung im Bilderfassungsbereich liegt.

Vorteilhafterweise verfügt die Vermessungseinrichtung über eine Auswerteeinheit, die auf der Grundlage der Aufnahmedaten eine Berechnung eines 3D-Datensatzes vornimmt. Damit kann sofort nach der Vermessung ein 3D-Datensatz erstellt werden.

In der Auswerteeinheit können vorteilhafterweise die Abstände der Rastpositionen der Verschiebeplatte auf der Rastplatte abgespeichert sein und die Auswerteeinheit nimmt auf der Grundlage der Aufnahmedaten und der bekannten Abstände eine Berechnung des 3D-Datensatzes vor.

Vorteilhafterweise wird eine Kalibration der Vermessungseinrichtung unter Verwendung eines Kalibrierkörpers vorgenommen, wobei der Kalibrierkörper eine bekannte Geometrie aufweist und die Kalibrierdaten in der Auswerteeinheit gespeichert und zur Auswertung der Aufnahmen verwendet werden. Die Vermessungseinrichtung besteht aus vielen Bauteilen, deren exakte Ausrichtung zueinander von Gerät zu Gerät durch Fertigungstoleranzen unterschiedlich sein kann. Eine Kalibration ist daher für die Genauigkeit der Auswertung von Vorteil.

Vorteilhafterweise wird bei der Kalibration die Ausrichtung der Bilderfassungseinheit zu den Verschieberichtungen der Verschiebeplatte bestimmt. Es ist möglich, dass die Verschieberichtungen und die Längsseiten des Bildaufnahmesensors der Bilderfassungseinheit nicht exakt parallel sind.

Eine Verschiebung der Verschiebplatte wird dann als schräge Verschiebung aufgenommen, was bei der Korrelation benachbarter Bilder anhand der Kalibrierdaten berücksichtigt wird.

Vorteilhaferweise wird bei der Kalibration die relative räumliche Ausrichtung der Rastplatte zur Bilderfassungseinheit bestimmt. Stimmen die Ebene der Rastplatte und die Ebene des Bildaufnahmesensors der Bildaufnahmeeinheit nicht überein, so bewirkt eine Verschiebung der Verschiebeplatte auf der Rastplatte zusätzlich eine Änderung des Abstands des zu vermessenden Objekts zur Bilderfassungseinheit.

Vorteilhafterweise wird bei der Kalibration die Veränderung des Bilderfassungsbereichs bei der Veränderung der Höhe der Bilderfassungseinrichtung über der Rastplatte bestimmt. Der Strahlengang der Bildaufnahmeeinheit einer Mund-Messkamera ist nicht zwangsläufig senkrecht zur Verschieberichtung der Bilderfassungseinheit bei einer Höhenänderung. Dadurch werden bei verschiedenen Höhen verschiedene Bildausschnitte aufgezeichnet.

Vorteilhafterweise sind an der Rastplatte Mittel zur Bestimmung der Position der Verschiebeplatte vorgesehen. Dies erleichtert die automatische Zuordnung von Aufnahmen, die in verschiedenen Positionen der Verschiebeplatte durchgeführt wurden.

Vorteilhafterweise ist die Oberfläche der Verschiebeplatte und/oder der Rastplatte mit einer gleitfähigen Schicht versehen. Damit wird das fehlerhafte Positionieren der Verschiebeplatte erschwert, außerdem wird die Bedienung leichter. Die Beschichtung kann darüber hinaus kratzfest sein, um eine lange Lebensdauer zu gewährleisten.

Besonders vorteilhaft ist es, wenn die Vermessungseinrichtung mit einem Anzeigegerät ausgestattet ist, auf dem die mit der Bildaufnahmeeinheit erzeugten Aufnahmen darstellbar sind. Die Anzeigeeinrichtung kann dann den aktuellen Bildausschnitt der Bildaufnahmeeinheit ständig anzeigen und der Bediener erhält die Möglichkeit, dass zu vermessende Objekt seinen Wünschen gemäß zu positionieren und die scharfe Schicht der Bildaufnahmeeinheit vor der Aufnahme einzustellen.

Vorteilhafterweise ist die verwendete Rastplatte austauschbar. Dann ist es möglich, die Rastplatte bei Beschädigung auszutauschen oder eine gegebenenfalls weiterentwickelte Rastplatte einzusetzen.

Vorteilhafterweise weist die Oberseite der Verschiebeplatte eine Befestigungsmöglichkeit für ein Zahnmodell auf und sind auf der Unterseite der Verschiebeplatte Erhöhungen vorhanden, auf denen die Verschiebeplatte steht. Eine solche Verschiebeplatte lässt sich abhängig von den Bedingungen, die dentale Vermessungseinrichtung bietet, frei positionieren. Die Verschiebeplatte kann aus der Vermessungseinrichtung heraus genommen werden und ein Zahnmodell darauf befestigt werden. Die Befestigung außerhalb der Vermessungseinrichtung lässt sich dann leichter bewerkstelligen.

Vorteilhafterweise ist die Befestigungsmöglichkeit schwenk-und kippbar. Dies ermöglicht einen breiteren Einsatzbereich, da ein zu vermessendes Zahnmodell aus verschiedenen Richtungen aufgenommen werden kann, wodurch Hinterschneidungen vermieden werden können.

Vorteilhafterweise sind auf der Oberseite der Verschiebeplatte Markierungen angebracht, deren Position der Position der Erhöhungen auf der Unterseite der Verschiebeplatte entspricht. Der Bediener erhält damit die Möglichkeit, die Position der Auflagepunkte leicht zu kontrollieren.

Vorteilhafterweise sind drei Erhöhungen vorgesehen, von denen zwei Erhöhungen so angeordnet sind, dass sie parallel zu einer Kante der Verschiebeplatte stehen. Dann existiert eine bevorzugte Verschieberichtung, die parallel zu der Kante der Verschiebeplatte ist.

Ein weiterer Gegenstand der Erfindung betrifft ein Verfahren zur 3D-Vermessung von Zahnmodellen. Zuerst wird eine erste Aufnahme des zu vermessenden Objekts erstellt, wobei sich die Verschiebeplatte in einer durch Rastmittel einer Rastplatte bestimmten ersten Position befindet. Danach wird in mindestens einer weiteren durch benachbarte Rastmittel bestimmten Position eine weitere Aufnahme erstellt. Dieser Schritt kann sooft wiederholt werden, bis das gesamte zu vermessende Objekt aufgenommen wurde. Anschließend wird ein 3D-Datensatz durch Auswertung der aus den mindestens zwei vorangegangen unterschiedlichen Aufnahmen unter Berücksichtigung der durch die Rastmittel vorgegebenen Positionen erstellt, wobei der Auswerteeinheit die Abstände der Rastposition der Verschiebeplatte auf der Rastplatte bekannt sind und die Aufnahmen werden von der Auswerteeinheit mit einem durch die Abstände benachbarter Rastposition der Verschiebeplatte auf der Rastplatte vorgegebenen Versatz zusammengefügt werden. Die Erstellung einer 3D-Vermessung von Zahnmodellen nach diesen Verfahren ist sehr sicher und mit geringer Fehleranfälligkeit durchführbar. Bei einem derartigen Verfahren ist die Korrelation von benachbarten Aufnahmen nicht mehr erforderlich, so dass keine Fehlerfortpflanzung existiert. Die zusammengesetzten Vermessungsdaten sind daher exakter als frei korrelierte Aufnahmen.

Vorteilhafterweise wird der aufgenommene Bildausschnitt vor der jeweiligen Aufnahme von einem Bediener an einem Anzeigegerät kontrolliert und der scharfe Bereich durch Höheneinstellungen der Aufnahmeeinheit so positioniert, dass der aufzunehmende Bereich scharf abgebildet wird. Dies gewährleistet eine scharfe Abbildung aller vermessungsrelevanten Daten und damit eine hohe Genauigkeit.

Vorteilhafterweise verfügt die Aufnahmeeinheit über Mittel zur Feststellung des scharfen Bereichs und über Mittel zum Verstellen des scharfen Bereichs, mit denen die Aufnahmeeinheit den scharfen Bereich automatisch einstellt. Dies gewährleistet immer die Einstellung des jeweils optimalen scharfen Bereichs und verhindert Fehlbedienungen.

Vorteilhafterweise vergleicht die Auswerteeinheit zur relativen Lagebestimmung der Aufnahmen die Einzelaufnahmen miteinander in einem Randbereich. Die Auswerteeinheit kann dadurch automatisch benachbart positionierte Aufnahmen erkennen und richtig zusammenfügen.

Vorteilhafterweise wird eine Kalibration der Vermessungseinrichtung vorgenommen, indem ein Kalibrationskörper auf einer Verschiebeplatte in mehreren Positionen aufgenommen wird und die Auswerteeinheit die aufgenommenen Positionen auswertet und daraus Kalibrierdaten ableitet und speichert. Damit wird eine optimale Vermessungsgenauigkeit auch bei vorhandenen Fertigungstoleranzen gewährleistet.

Vorteilhafterweise wird eine bei der Höhenverstellung der Bildaufnahmeeinrichtung auftretende Verschiebung des Bildausschnitts von der Auswerteeinheit automatisch korrigiert. Die Auswerteeinheit benutzt dazu die bei der Kalibration der Vermessungseinrichtung festgestellte Verschiebungsrichtung, die bei der Aufnahme zweier Bilder aus unterschiedlichen Höhen den Versatz der Bilder angibt, um gleiche Bildteile zweier an benachbarten Positionen aufgenommener Bilder entlang der Verschieberichtung so zu verschieben, dass beide Aufnahmen im Randbereich bestmöglich aufeinander passen. Dies gleicht einen gegebenenfalls vorhandenen vertikalen Versatz der Aufnahmen zueinander bei der Aufnahme aus unterschiedlichen Höhen aus.

Vorteilhafterweise werden die Einzelaufnahmen zu einem gemeinsamen Datensatz zusammengesetzt. Das Resultat ist dann ein alle relevanten Bereiche umfassender Datensatz, der so bestmöglich weiterverarbeitet werden kann.

Eine erfindungsgemäße Vermessungseinrichtung wird anhand der Zeichnung erläutert.

Es zeigen die:
- Fig. 1: eine schematische Darstellung einer erfindungsgemäßen dreidimensionale Vermessungseinrichtung, die
- Fig. 2: eine Draufsicht auf eine Verschiebeplatte mit einer verkippbaren Befestigungseinheit und die dazugehörige Rastplatte, die
- Fig. 3: eine Draufsicht auf die Verschiebeplatte und die dazugehörige Rastplatte, die
- Fig. 4: eine Schrägansicht der Unterseite der Verschiebeplatte, die
- Fig. 5: die Rastplatte ohne Verschiebeplatte in Schrägansicht, die
- Fig. 6: die Rotationsvermessung von Objekten in der Vermessungseinrichtung, die
- Fig. 7: die Rastplatte und eine Verschiebeplatte zur Kalibration des Systems, die
- Fig. 8: die zu kalibrierenden Freiheitsgrade der Vermessungseinrichtung, die
- Fig. 9: die Darstellung der Vermessungsbereiche anhand eines Zahnmodells, und die
- Fig. 10: den Ablauf einer Vermessung als Struktogramm.

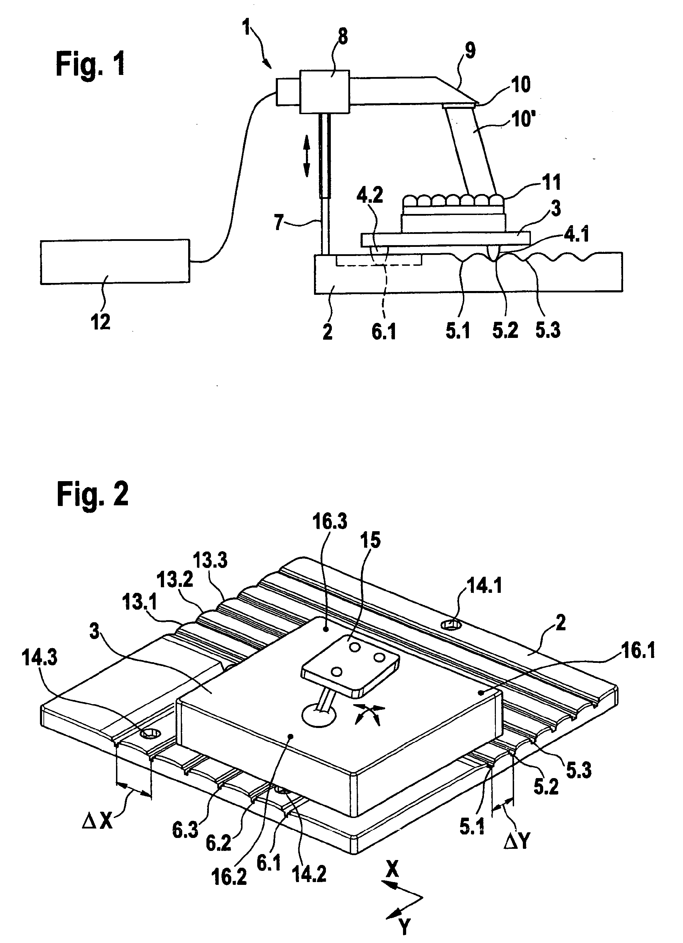

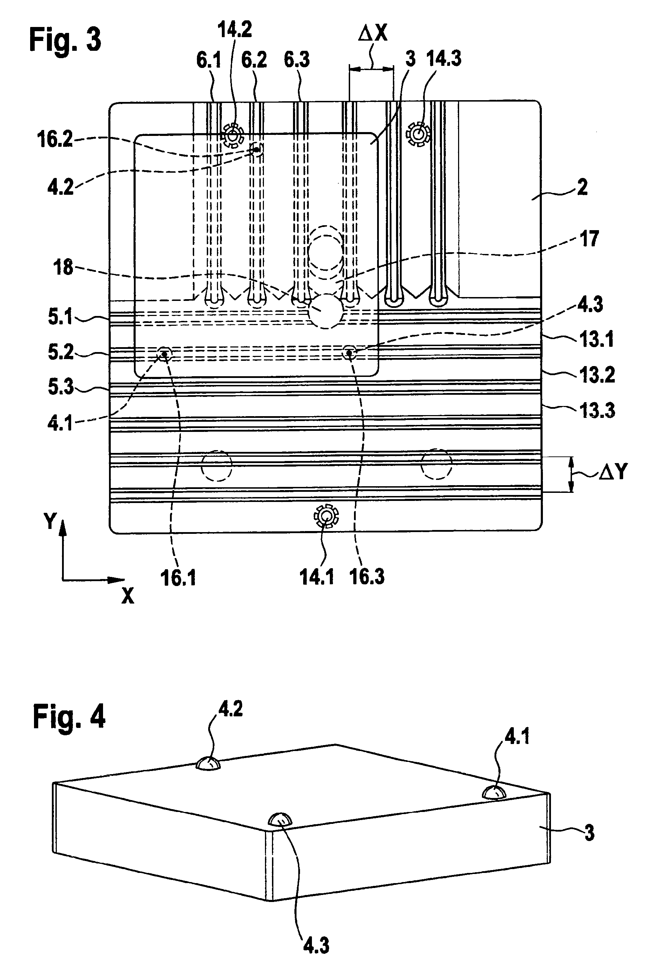

Die **Fig. 1** zeigt eine erfindungsgemäße Vermessungseinrichtung 1 in schematischer Darstellung. Die Vermessungseinrichtung 1 umfasst eine Rastplatte 2, deren Gestaltung in den Fig. 2 und Fig. 3 genauer erläutert wird. Auf der Rastplatte 2 befindet sich eine Verschiebeplatte 3, die mit Erhöhungen 4.1, 4.2, 4.3 (nicht dargestellt) auf der Rastplatte 2 aufliegt. Die Erhöhungen 4.1 und 4.3 rasten dabei in einer der Nuten in X-Richtung 5.1, 5.2, 5.3, und die Erhöhung 4.2 in eine der Nuten in Y-Richtung 6.1, 6.2, 6.3 oder weitere ein. Dargestellt ist hier ein Einrasten der Erhöhungen 4.1 und 4.3 in die Vertiefungen 5.2 und der Erhöhung 4.2 in 6.1. Die Rastplatte 2 ist mit einer kratzfesten und gleitfähigen Oberfläche beschichtet.

An der Rastplatte (2) ist weiterhin ein höhenverstellbares Stativ 7 mit einer Halterung 8 für eine Messkamera 9 vorgesehen, die dem Prinzip nach einer Mund-Messkamera entspricht. Das Objektiv der Messkamera 9 ist mit einem Lichtfilter 10 versehen, um eine Optik der Messkamera vor Umgebungsbeleuchtung zu schützen. Das Lichtfilter 10 schützt die Optiken durch die exponierte Lage außerdem vor mechanischer Beschädigung. Die Messkamera 9 ist auf die Verschiebeplatte 3 mit dem darauf befestigten, zu vermessenden Objekt 11 ausgerichtet, wobei der Strahlengang 10' telezentrisch ist und eine leichte Neigung von bis zu 5 Grad gegenüber der Vertikalen aufweist, wie dies bei den verwendeten Mund-Messkameras üblich ist.

Die Messkamera 9 ist mit einer Auswerteeinheit 12 verbunden, die die Vermessungsdaten der Messkamera aufnimmt, speichert und auswertet.

Die **Fig. 2** verdeutlicht die Ausgestaltung der Rastplatte 2 sowie der Verschiebeplatte 3. Auf der Rastplatte 2 befinden sich zwei Gruppen von jeweils parallelen, gleich beabstandeten Nuten 5.1, 5.2, 5.3 sowie 6.1, 6.2, 6.3, die im Wesentlichen aufeinander senkrecht stehen. Die Übergänge der Nuten, zum Beispiel von 5.1 nach 5.2, hier 13.1, sind im Wesentlichen konvex gestaltet. Die Rastplatte 2 kann mittels der Bohrungen 14.1, 14.2, 14.3 auf einem Tisch befestigt werden. Die Abstände ΔX der Nuten in X-Richtung 6.1, 6.2, 6.3 und ΔY der Nuten in Y-Richtung 5.1, 5.2, 5.3 sind an den Bilderfassungsbereich der Messkamera 9 angepasst. Das erfasste Bild hat hat im vorliegenden Fall eine Ausdehnung von 17 mm in X-Richtung und 14 mm in Y-Richtung. Es ist eine Überlappung von 2mm an jedem Randbereich vorgesehen, sodaß ΔX hier 15 mm und ΔY 12 mm beträgt.

Auf der Verschiebeplatte 3 ist eine schwenkbare Halterung 15 angebracht, die es ermöglicht, ein darauf befestigtes zu vermessends Objekt 11 sowohl in beliebiger Höhe als auch in beliebiger Richtung und Neigung zu befestigen.

Des Weiteren sind Markierungen 16.1, 16.2, 16.3 auf der Oberseite der Verschiebeplatte 3 angebracht, die die Position der darunterliegenden Auflagepunkte 4.1, 4.2, 4.3 (nicht dargestellt) wiedergeben.

In **Fig. 3** ist die Rastplatte 2 und die Verschiebeplatte 3 in einer Draufsicht dargestellt. Die Erhöhungen 4.1 und 4.3 befinden sich hier in der Nut 5.2, die Erhöhung 4.2 befindet sich in der Nut 6.2. Nach dem Fertigstellen einer Aufnahme kann die Verschiebeplatte 3 nun von dieser ersten Position in eine weitere exakt bestimmte Position verschoben werden, beispielsweise in Y-Richtung durch das Bringen der Erhöhungen 4.1 und 4.3 in die Nut 5.1. Während dieser Verschiebung gleitet die Erhöhung 4.2 die ganze Zeit in der Nut 6.2. Auch eine Verschiebung in X-Richtung ist möglich, dann gleiten die Erhöhungen 4.1 und 4.3 in der Nut 5.2, während die Erhöhung 4.2 in die Nut 6.3 gebracht wird.

Es entsteht so bei Betrachtung aller möglichen Positionen der Verschiebeplatte eine Matrix mit gleichen Abständen in X- sowie in Y-Richtung.

An der Rastplatte 2 der Verschiebeplatte befindet sich eine Bohrung 16, die zur Aufnahme eines Halters dient. Unterhalb der Rastplatte befindet sich eine schräg nach oben weisende drehbare Halterung, in der ein Objekt, das von der Messkamera 9 vollständig erfassbar ist gehaltert und von allen Seiten vermessen werden kann.

In **Fig. 4** ist die Verschiebeplatte 3 von der Unterseite her gesehen dargestellt. Die Erhöhungen 4.1, 4.2 und 4.3 sind kugelförmig gestaltet. Zusammen mit konvexen Übergängen zwischen den Nuten ist eine fehlerhafte Positionierung so nahezu ausgeschlossen.

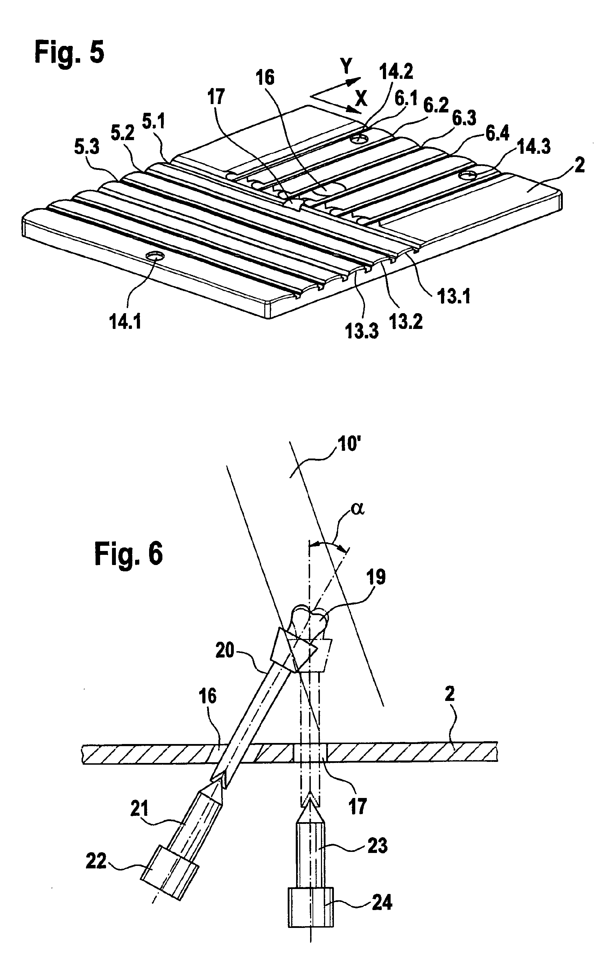

Die **Fig. 5** zeigt die erfindungsgemäße Rastplatte in einer dreidimensionalen Schrägansicht. Auf der Rastplatte sind zwei Durchbrechungen 16, 17 angeordnet. Diese Durchbrechungen 16, 17 dienen zur Aufnahme eines Halters, an den ein zu vermessendes Zahnmodell 18 angebracht werden kann. Die Durchbrechungen sind so angeordnet, dass mit der Auswahl eines passend gestalteten Halters der Zahn in demselben Strahlengang der Bildaufnahmeeinrichtung positioniert ist. Die Durchbrechungen 16, 17 sind auf der Erhöhung zwischen den Nuten 6.3 und 6.4 angeordnet. Dadurch ist eine Fehlpositionierung der Verschiebeplatte durch das Hereinrutschen des Auflagepunktes 4.2 in eine der Durchbrechungen 16, 17 nicht mehr möglich.

Die Durchbrechung 16 dient der Halterung eines Zahns unter einem vorgegeben Winkel α, der hier 30° beträgt (Fig. 6). Die Halterung ist drehbar, so dass der Zahn von allen Seiten vermessen werden kann. Die Durchbrechung erlaubt es den Zahn exakt senkrecht zur Oberfläche der Rastplatte (2) auszurichten, so dass die Vermessung von eventuell auf der Oberseite des Zahnmodells 18 vorhandenen Kavitäten möglich ist.

Die Funktion der Durchbrechung 16, 17 und die Positionierung von Objekten, die mittels Rotationsvermessung vermessen werden sollen, ist in **Fig. 6** dargestellt. Ein Objekt 19 ist auf einem Halter 20 befestigt, der auf einer unterhalb der Rastplatte 2 befindlichen Spindel 21 eines Motors 22 aufgesteckt ist. Das zu vermessend Objekt 19, hier ein Zahn, befindet sich im Strahlengang 10' der Bilderfassungseinrichtung 9. Der Zahn 19 wird mittels des Motors 22 schrittweise um einen bestimmten Winkelbetrag, beispielsweise 45° gedreht und eine weitere Aufnahme wird vorgenommen. Durch den Winkel α von 30° wird sichergestellt, dass der Zahn 19 von allen Seiten vollständig ohne Hinterschneidungen erfasst wird. Sollten sich auf der Oberseite des Zahns 19 noch Kavitäten befinden, kann der Halter durch die Durchbrechung 17 auf die Spindel 23 mit dem Motor 24 aufgesteckt werden. Somit können Aufnahmen der Oberseite das Zahns 19 aus verschiedenen Richtungen erstellt werden, die solche Kavitäten erfasst.

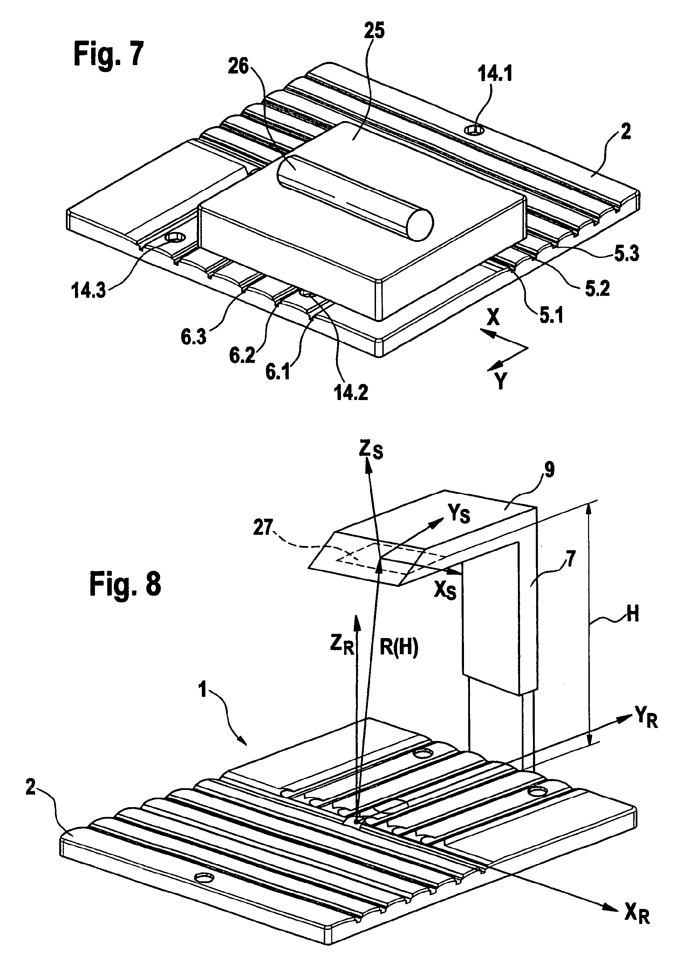

Die **Fig. 7** zeigt eine Kalibriereinrichtung 25 mit einem Kalibrierkörper 26, die dazu dient, die Vermessungseinrichtung zu kalibrieren. Die Kalibriereinrichtung 25 weist dazu auf der Unterseite ähnlich wie die Verschiebeplatte drei nicht das gestellte Erhöhungen auf, die in die Vertiefungen 5.1, 5.2, 5.3 und 6.1, 6.2, 6.3 der Rastplatte 2 eingreifen. Der Kalibrierkörper 26 ist ein sehr präzise gefertigter Zylinder, der fest auf der Kalibriereinheit 25 aufgebracht ist. Die Auswerteeinheit 12 ist in der Lage, aus den Messdaten die Achse des Kalibrierkörpers 26 zu erkennen.

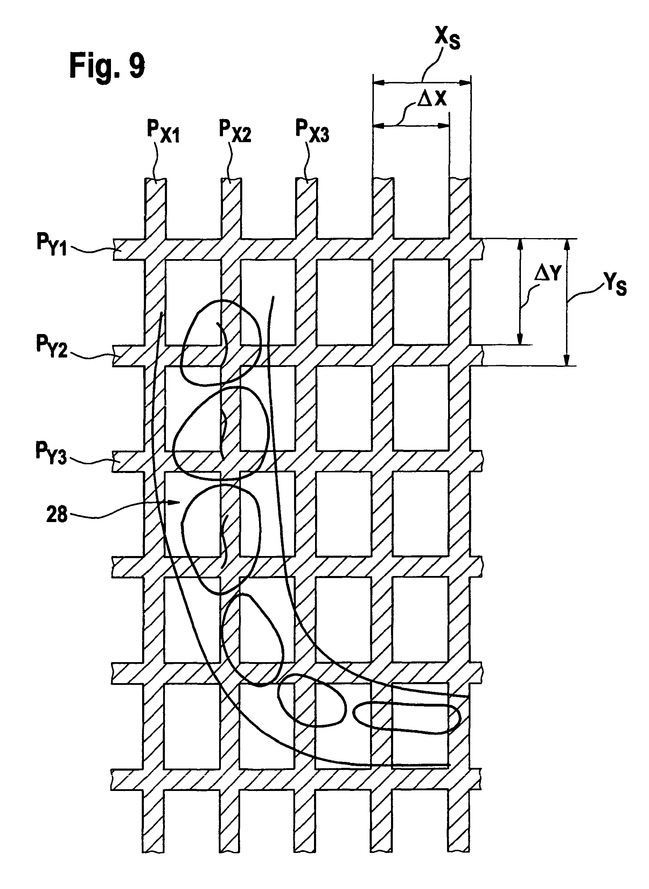

Die Vorgehensweise bei der Kalibrierung wird im Nachfolgenden anhand der **Fig. 8** und der **Fig. 9** erläutert. Die Rastplatte 2 der dreidimensionalen Vermessungseinrichtung 1 hat eine im Raum fest vorgegebene Ausrichtung, die mit X_{R}, Y_{R} und Z_{R} dargestellt ist. Die Koordinaten sind so gewählt, dass X_{R} in Richtung der einen Verschieberichtung und Y_{R} in Richtung der anderen Verschieberichtung zeigt, wobei Z_{R} die Richtung der Flächennormale der Rastplatte 2 angibt. Darüber hinaus existiert ein weiteres Koordinatensystem X_{S}, Y_{S}, Z_{S} des Sensors 27, das gegenüber dem Koordinatensystem der Rastplatte 2 verdreht ist. Die Richtungen X_{S} und Y_{S} sind parallel zu den Kanten des Bildsensors gewählt, Die Richtung Z_{S} bestimmt den Flächennormalenvektor des Bildsensors 27. Die beiden Koordinatensysteme X_{R}, Y_{R}, Z_{R} und X_{S}, Y_{S}, Z_{S} sind durch Fertigungstoleranzen gegeneinander verdreht und verkippt.

Die erste Aufgabe der Kalibrierung besteht darin, die relative Lage der beiden Koordinatensysteme zueinander zu bestimmen. Dazu wird die in Fig. 7 dargestellte Korrelationseinheit 25 in vier verschiedenen Positionen, die jeweils die Extrempositionen der Kalibriereinheit 25 auf der Rastplatte 2 darstellen, vermessen. Aus der Änderung der Achslage der Achse des Korrelationskörpers 26 kann die Auswerteeinheit zum einen die relative Ausrichtung der beiden Koordinatensysteme zueinander und zum anderen die Verschieberichtungen X_{R} und Y_{R} der Verschiebeplatte 3 bezüglich der Lage des Bildsensors, gegeben durch X_{S} und Y_{S}, bestimmen.

Im Anschluss wird eine weitere Kalbration vorgenommen, die die absolute Lage der beiden Koordinatensysteme zueinander in Abhängigkeit von der Höhe H der Bildaufnahmeeinheit von der Rastplatte bestimmt. Der Vektor R(H) ist, zusammen mit der durch den schrägen Strahlengang 10' verursachten Bildversatz für den absoluten Bildversatz in Abhängigkeit der Höhenänderung verantwortlich.

Zur Feststellung dieser Abhängigkeit wird die Korrelationseinheit 25 in zwei zueinander senkrecht stehenden Positionen auf die Rastplatte 2 gesetzt und in jeweils zwei verschiedenen Höhen von der Bildaufnahmeeinrichtung 9 vermessen. Aus den vier Aufnahmen kann der Verschiebevektor R(H) eindeutig bestimmt werden.

Die **Fig. 9** zeigt die Vermessung eines Zahnmodells 28. Das Zahnmodell 28 wird zusammen mit der Verschiebeplatte 3 auf der Rastplatte verschoben und kann dabei nur vorgegebene Positionen einnehmen, die hier mit P_{X1}, P_{X2}, P_{X3} und P_{Y1}, P_{Y2}, P_{Y3} dargestellt sind. Die Abstände ΔX in X-Richtung und ΔY in Y-Richtung sind durch die Abstände der Nuten vorgegeben. Der Bildaufnahmebereich XS, YS des Sensors ist größer als die Rastung. Dadurch entstehen bei benachbarten Aufnahmen Überlappungsbereiche, die hier schraffiert dargestellt sind. Diese Überlappungsbereiche werden von der Auswerteeinheit 12 dazu verwendet, die entsprechenden Einzelaufnahmen zusammen zu setzten.

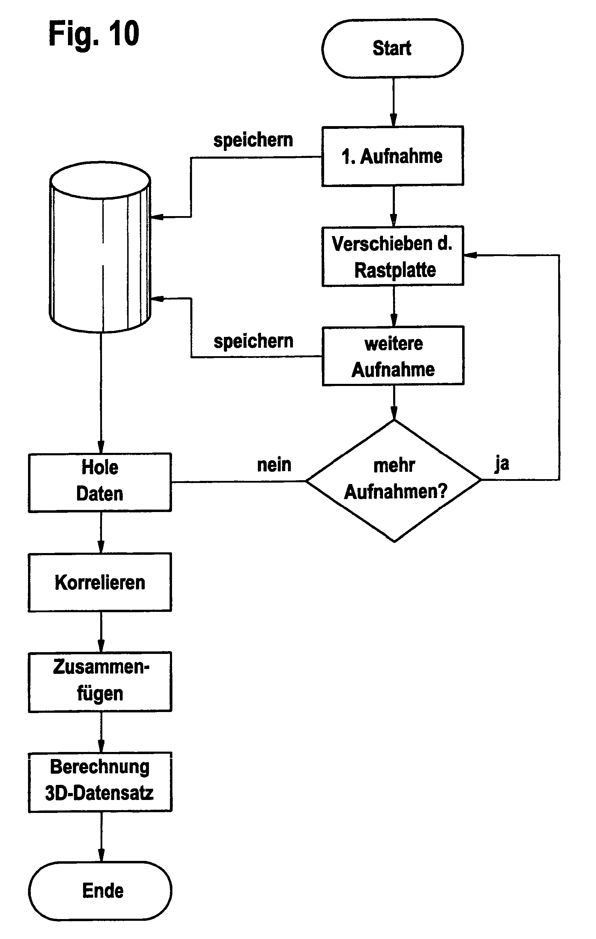

Die **Fig. 10** zeigt das erfindungsgemäße Verfahren anhand eines Ablaufdiagramms in einer ersten Position der Verschiebeplatte wird eine erste Aufnahme vorgenommen. Die Aufnahmedaten werden dabei auf einem Datenträger gespeichert, der sich in der Auswerteeinheit befindet. Danach wird die Verschiebeplatte in X- oder Y-Richtung um eine Nut verschoben, wie es in der vorangegangenen Figurenbeschreibung dargestellt wurde. Danach wird in dieser weiteren Position eine neue Aufnahme vorgenommen. Die Daten werden wiederum in den Datenspeicher der Auswerteeinheit gespeichert. Sind noch weitere Aufnahmen vorzunehmen, so wiederholt sich der letzte Schritt ab dem Verschieben der Verschiebeplatte, solange bis das gesamte zu vermessende Objekt vermessen wurde. Nach der Beendigung des Aufnahmevorgangs holt die Auswerteeinheit 12 die Daten aus dem Speicher, korreliert die Überlappungsbereiche der einzelnen Aufnahmen und fügt die Aufnahmen unter Berücksichtigung des bekannten Abstands der Vertiefungen 5.1, 5.2 sowie 6.1, 6.2 zusammen und berechnet daraus einen vollständigen 3D-Datensatz.

### Bezugszeichenliste

- 1: Vermessungseinrichtung
- 2: Rastplatte
- 3: Verschiebeplatte
- 4.1, 4.2, 4.3: Erhöhungen
- 5.1, 5.2, 5.3: Nuten in X-Richtung
- 6.1, 6.2, 6.3: Nuten in Y-Richtung
- 7: Stativ
- 8: Halterung
- 9: Messkamera
- 10: Objektivschutz
- 10': Strahlengang
- 11: zu vermessendes Objekt
- 12: Auswerteeinheit
- 13.1, 13.2, 13.3: Übergang
- 14.1, 14.2, 14.3: Befestigungsbohrungen
- 15: schwenkbare Halterung
- 16.1, 16.2, 16.3: Markierung
- 17: Aufnahme für drehbare Vermessung
- 18: Aufnahme für senkrechte Vermessung
- 19: Zahn
- 20: Halter
- 21: Spindel
- 22: Motor
- 23: Spindel
- 24: Motor
- 25: Kalibriereinrichtung
- 26: Korrelationskörper
- 27: Sensor
- 28: Zahnmodell

## Patentansprüche

1. Vermessungseinrichtung (1) zur 3D-Vermessung von Zahnmodellen, aufweisend eine Aufnahmevorrichtung (9) und eine Positionierungseinrichtung(2, 3), wobei die Positionierungseinrichtung (2, 3) eine bezüglich der Aufnahmevorrichtung positionierbare Platte (3), auf der das zu vermessende Zahnmodell befestigbar ist, und eine bezüglich der Aufnahmevorrichtung feststehende Grundplatte (2) aufweist, **dadurch gekennzeichnet, dass** die positionierbare Platte (3) als Verschiebeplatte (3) ausgebildet ist und erste Rastmittel (4.1, 4.2, 4.3) enthält, dass die Grundplatte als Rastplatte (2) ausgeführt ist und dass an der Rastplatte (2) zweite Rastmittel (5.1, 5.2, 5.3, 6.1, 6.2, 6.3) vorgesehen sind, die mit den ersten Rastmitteln (4.1, 4.2, 4.3) so zusammenwirken, dass die Verschiebeplatte (3) eine von mehreren vorgegebenen Positionen zur Rastplatte (2) einnehmen kann und in der ausgewählten Position einrastet, wobei die Aufnahmevorrichtung (9) als Bilderfassungseinheit (9) ausgestattet ist und wobei die Abstände benachbarter Rastmittel (5.1 bis 5.3, 6.1 bis 6.3) der Rastplatte (2) in X- und in Y-Richtung kleiner oder gleich dem Bilderfassungsbereich der Bilderfassungseinheit (9) und größer als der halbe Bilderfassungsbereich sind.

2. Vermessungseinrichtung nach Anspruch 1, **dadurch gekennzeichnet, dass** die Aufnahmevorrichtung (9) als Mund-Messkamera (9) mit vorzugsweise telezentrischem Beobachtungsstrahlengang ausgestaltet ist.

3. Vermessungseinrichtung nach Anspruch 2, **dadurch gekennzeichnet, dass** sich die in benachbarten Positionen aufgenommenen Bildbereiche um mindestens 1/10 des Bilderfassungsbereichs überlappen.

4. Vermessungseinrichtung nach einem der Ansprüche 1 bis 3, **dadurch gekennzeichnet, dass** die einen Rastmittel (4.1, 4.2, 4.3) als Erhöhungen und die anderen Rastmittel (5.1, 5.2, 5.3, 6.1, 6.2, 6.3) als Vertiefungen ausgebildet sind, wobei die Erhöhungen (4.1, 4.2, 4.3) vorzugsweise im Wesentlichen eine konvexe Auflagefläche aufweisen und die Vertiefungen (5.1, 5.2, 5.3, 6.1, 6.2, 6.3) als Nuten ausgebildet sind, wobei der Übergang zwischen zwei Nuten (5.1, 5.2, 5.3, 6.1, 6.2, 6.3) vorzugsweise konvex gestaltet ist.

5. Vermessungseinrichtung nach Anspruch 4, **dadurch gekennzeichnet, dass** die zweiten Rastmittel (5.1, 5.2, 5.3, 6.1, 6.2, 6.3) in einen ersten Bereich mit parallelen, gleich beabstandeten Nuten und in einen zweiten Bereich mit parallelen, gleich beabstandeten Nuten aufgeteilt sind, wobei die Nuten des ersten Bereichs im Wesentlichen senkrecht zu den Nuten des zweiten Bereichs stehen.

6. Vermessungseinrichtung nach einem der Ansprüche 1 bis 5, **dadurch gekennzeichnet, dass** die Rastplatte (2) mindestens eine Durchbrechung (17, 18) zur Befestigung eines in X- wie in Y-Richtung verschiebungssfest zu vermessenden Objektes (19) über ein Befestigungsmittel enthält, wobei das Befestigungsmittel (21, 23) so ausgebildet ist, dass das Objekt im selben Bilderfassungsbereich wie das verschiebbar zu vermessende Objekt (11) liegt.

7. Vermessungseinrichtung nach einem der Ansprüche 1 bis 6, **dadurch gekennzeichnet, dass** eine Auswerteeinheit (12) vorgesehen ist, die auf der Grundlage der Aufnahmedaten eine Berechnung eines 3D-Datensatzes vornimmt und dass weiterhin eine Kalibration der Vermessungseinrichtung (1) unter Verwendung eines Kalibrierkörpers (25, 26) mit bekannter Geometrie erfolgt und die aus der Kalibration ermittelten Kalibrierdaten in der Auswerteeinheit (12) bei der Auswertung der Aufnahmen verwendet werden.

8. Vermessungseinrichtung nach Anspruch 7, **dadurch gekennzeichnet, dass** bei der Kalibration die Ausrichtung der Bilderfassungseinheit (9) zu den Verschieberichtungen X, Y der Verschiebeplatte (3) sowie vorzugsweise die relative räumliche Ausrichtung der Rastplatte (2) zur Bilderfassungseinheit (9) sowie vorzugsweise die Veränderung des Bilderfassungsbereichs bei der Veränderung der Höhe der Bilderfassungseinrichtung (9) über der Rastplatte (2) bestimmt ist.

9. Vermessungseinrichtung nach einem der Ansprüche 1 bis 8, **dadurch gekennzeichnet, dass** auf der Oberseite der Verschiebeplatte (3) eine schwenk- und kippbare Befestigungsmöglichkeit (15) für ein Zahnmodell (11) vorgesehen ist, dass auf der Unterseite der Verschiebeplatte (3) Erhöhungen (4.1, 4.2, 4.3) vorhanden sind, auf denen die Verschiebeplatte (3) steht und dass die Verschiebeplatte (3) aus der Vermessungseinrichtung herausnehmbar und frei positionierbar ist.

10. Vermessungseinrichtung nach Anspruch 9, **dadurch gekennzeichnet, dass** auf der Oberseite der Verschiebeplatte (3) Markierungen (16.1, 16.2, 16,3) angebracht sind, deren Position der Position der Erhöhungen (4.1, 4.2, 4.3) auf der Unterseite entspricht.

11. Vermessungseinrichtung nach Anspruch 9 oder 10, **dadurch gekennzeichnet, dass** drei Erhöhungen (4.1, 4.2, 4.3) vorgesehen sind, von denen zwei Erhöhungen (4.1, 4.3) so positioniert sind, dass sie parallel zu einer Kante der Verschiebeplatte (3) stehen.

12. Verfahren zur 3D-Vermessung von Zahnmodellen, wobei ein zu vermessendes Objekt (11) auf einer positionierbaren Platte (3) befestigt wird und das zu vermessende Objekt (11) von einer Bilderfassungseinheit (9) in mehreren Aufnahmen erfasst wird und zur Erstellung eines 3D-Datensatzes des Zahnmodells (11) eine Berechnung durch eine Auswerteeinheit (12) vorgenommen wird, **gekennzeichnet durch** folgende Schritte:
- die Erstellung einer ersten Aufnahme des zu vermessenden Objekts (11) an einer **durch** Rastmittel (5.1, 5.2, 5.3, 6.1, 6.2, 6.3) einer mit der Bilderfassungseinheit verbundenen Rastplatte (2) bestimmten Position,
- die Positionierung der als Verschiebeplatte ausgebildeten Platte (3) in mindestens einer weiteren, **durch** benachbarte Rastmittel (5.1, bis 5.3, 6.1 bis 6.3) der Rastplatte (2) vorgegebenen Position und die Erstellung einer weiteren Aufnahme je Position und
- die Erstellung eines 3D-Datensatzes **durch** Auswertung der aus den mindestens zwei unterschiedlichen, **durch** benachbarte Rastmittel (5.1, bis 5.3, 6.1 bis 6.3) genau vorgegebenen Positionen erstellten Aufnahmen, wobei der Auswerteeinheit (12) die Abstände der Rastpositionen der Verschiebeplatte (3; 25) auf der Rastplatte (2) bekannt sind und die Auswerteeinheit (12) die Aufnahmen mit einem **durch** die Abstände der Rastpositionen der Verschiebeplatte (3; 25) auf der Rastplatte (2) vorgegebenen Versatz zusammenfügt.

13. Verfahren nach Anspruch 12, **dadurch gekennzeichnet, dass** die Aufnahmeeinheit (9) den scharfen Bereich durch Mittel zur Feststellung des scharfen Bereichs und Mittel zum Verstellen des scharfen Bereichs automatisch einstellt.

14. Verfahren nach Anspruch 12 oder 13, **dadurch gekennzeichnet, dass** die Auswerteeinheit (12) zur relativen Lagebestimmung der Aufnahmen zueinander die Einzelaufnahmen miteinander in einem Randbereich der Aufnahmen vergleicht.

15. Verfahren nach einem der Ansprüche 12 bis 14, **dadurch gekennzeichnet, dass** eine Kalibration der Vermessungseinrichtung (1) vorgenommen wird, indem ein Kalibrationskörper (26) auf einer Verschiebeplatte (25) in mehreren Positionen aufgenommen wird und die Auswerteeinheit (12) die aufgenommenen Positionen auswertet und Kalibrierdaten abspeichert.

16. Verfahren nach einem der Ansprüche 12 bis 15, **dadurch gekennzeichnet, dass** eine bei der Höhenverstellung der Bildaufnahmeeinrichtung (9) auftretende Verschiebung des Bildausschnitts von der Auswerteeinheit (12) automatisch korrigiert wird, indem die bei der Kalibration der Vermessungseinrichtung (1) festgestellte Verschiebungsrichtung dazu benutzt wird, gleiche Bildteile zweier an benachbarten Positionen aufgenommener Bilder entlang der Verschiebungsrichtung so zu verschieben, dass beide Aufnahmen im Randbereich bestmöglich aufeinander passen.

## Claims

1. Measuring device (1) for the 3D measurement of tooth models, having a recording apparatus (9) and a positioning device (2, 3), the positioning device (2, 3) having a plate (3) which can be positioned with reference to the recording apparatus and on which the tooth model to be measured can be fastened, and a base plate (2) fixed with reference to the recording apparatus, **characterized in that** the positionable plate (3) is designed as a displaceable plate (3) and includes first latching means (4.1, 4.2, 4.3), **in that** the base plate is designed as a latching plate (2), and **in that** there are provided on the latching plate (2) two latching means (5.1, 5.2, 5.3, 6.1, 6.2, 6.3) which cooperate with the first latching means (4.1, 4.2, 4.3) such that the displaceable plate (3) can assume one of a number of prescribed positions relative to the latching plate (2) and latches tight in the selected position, the recording apparatus (9) being equipped as an image acquisition unit (9), and the spacings of neighbouring latching means (5.1 to 5.3, 6.1 to 6.3) of the latching plate (2) in the X- and in the Y-directions being smaller than or equal to the image acquisition range of the image acquisition unit (9) and greater than half the image acquisition range.

2. Measuring device according to Claim 1, **characterized in that** the recording apparatus (9) is configured as a mouth measurement camera (9) with a preferably telecentric observation beam path.

3. Measuring device according to Claim 2, **characterized in that** the image areas recorded in neighbouring positions overlap by at least 1/10 of the image acquisition range.

4. Measuring device according to one of Claims 1 to 3, **characterized in that** some of the latching means (4.1, 4.2, 4.3) are designed as elevations and the other latching means (5.1, 5.2, 5.3, 6.1, 6.2, 6.3) are designed as depressions, the elevations (4.1, 4.2, 4.3) preferably substantially having a convex bearing surface, and the depressions (5.1, 5.2, 5.3, 6.1, 6.2, 6.3) being designed as grooves, the transition between two grooves (5.1, 5.2, 5.3, 6.1, 6.2, 6.3) preferably being of convex shape.

5. Measuring device according to Claim 4, **characterized in that** the second latching means (5.1, 5.2, 5.3, 6.1, 6.2, 6.3) are split into a first region with parallel, equally spaced grooves, and into a second region with parallel, equally spaced grooves, the grooves of the first region being substantially perpendicular to the grooves of the second region.

6. Measuring device according to one of Claims 1 to 5, **characterized in that** the latching plate (2) includes at least one cutout (17, 18) for fastening, via fastening means, an object (19) to be measured in a fashion secure against displacement in the X- and the Y- directions, the fastening means (21, 23) being designed such that the object lies in the same image acquisition range as the object (11) to be measured displaceably.

7. Measuring device according to one of Claims 1 to 6, **characterized in that** an evaluation unit (12) is provided which undertakes to calculate a 3D data record on the basis of the recorded data, and **in that**, furthermore, the measuring device (1) is calibrated by using a calibration body (25, 26) of known geometry and the calibration data determined from the calibration are used in the evaluation unit (12) when evaluating the pictures.

8. Measuring device according to Claim 7, **characterized in that** in the calibration the alignment of the image acquisition unit (9) relative to the displacement directions X, Y of the displaceable plate (3) is determined, and the relative spatial alignment of the latching plate (2) relative to the image acquisition unit (9) is preferably determined, and the variation in the image acquisition range with the variation in the height of the image acquisition device (9) above the latching plate (2) is preferably determined.

9. Measuring device according to one of Claims 1 to 8, **characterized in that** a pivotable and tiltable fastening facility (15) for a tooth model (11) is provided on the topside of the displaceable plate (3), **in that** elevations (4.1, 4.2, 4.3) on which the displaceable plate (3) stands are provided on the underside of the displaceable plate (3), and **in that** the displaceable plate (3) can be removed from the measuring device and be freely positioned.

10. Measuring device according to Claim 9, **characterized in that** applied to the topside of the displaceable plate (3) are marks (16.1, 16.2, 16.3) whose position corresponds to the position of the elevations (4.1, 4.2, 4.3) on the underside.

11. Measuring device according to Claim 9 or 10, **characterized in that** three elevations (4.1, 4.2, 4.3) are provided of which two elevations (4.1, 4.3) are positioned such that they are parallel to an edge of the displaceable plate (3).

12. Method for the 3D measurement of tooth models, an object (11) to be measured being fastened on a positionable plate (3), and the object (11) to be measured being acquired in a number of pictures by an image acquisition unit (9), and a calculation being undertaken by an evaluation unit (12) in order to compile a 3D data record of the tooth model (11), **characterized by** the following steps:
- taking a first picture of the object (11) to be measured at a position determined by latching means (5.1, 5.2, 5.3, 6.1, 6.2, 6.3) of a latching plate (2) connected to the image acquisition unit,
- positioning the plate (3) designed as a displaceable plate in at least one further position prescribed by neighbouring latching means (5.1 to 5.3, 6.1 to 6.3) of the latching plate (2), and taking a further picture per position, and
- compiling a 3D data record by evaluating the pictures taken from the at least two different positions exactly prescribed by neighbouring latching means (5.1 to 5.3, 6.1 to 6.3), the spacings of the latching positions of the displaceable plate (3; 25) on the latching plate (2) being known to the evaluation unit (12), and the evaluation unit (12) combining the pictures with an offset prescribed by the spacings of the latching positions of the displaceable plate (3; 25) on the latching plate (2).

13. Method according to Claim 12, **characterized in that** the recording unit (9) automatically sets the sharp region by means for establishing the sharp region and means for adjusting the sharp region.

14. Method according to Claim 12 or 13, **characterized in that** the evaluation unit (12) compares the individual pictures with one another in an edge region of the pictures for the purpose of determining the positions of the pictures relative to one another.

15. Method according to one of Claims 12 to 14, **characterized in that** a calibration of the measuring device (1) is undertaken by recording a calibration body (26) in a number of positions on a displaceable plate (25), and using the evaluation unit (12) to evaluate the recorded positions and store calibration data.

16. Method according to one of Claims 12 to 15, **characterized in that** a displacement, occurring during the height adjustment of the image recording device (9), of the image section is corrected automatically by the evaluation unit (12) by using the displacement direction established during the calibration of the measuring device (1) to displace the same image parts of two images recorded at neighbouring positions along the displacement direction such that the two pictures match one another as best as possible in the edge region.

## Revendications

1. Dispositif de mesure (1) pour la mesure 3D de modèles de dent, présentant un dispositif de logement (9) et un dispositif de positionnement (2, 3), le dispositif de positionnement (2, 3) présentant une plaque (3) positionnable par rapport au dispositif de logement, sur laquelle le modèle de dent à mesurer est fixé, et une plaque de base (2) fixe par rapport au dispositif de logement, **caractérisé en ce que** la plaque (3) positionnable est conçue comme plaque coulissante (3) et comporte des premiers moyens d'encliquetage (4.1, 4.2, 4.3), **en ce que** la plaque de base est conçue comme plaque d'encliquetage (2) et **en ce que** sur la plaque d'encliquetage (2) sont prévus des seconds moyens d'encliquetage (5.1, 5.2, 5.3, 6.1, 6.2, 6.3) qui coopèrent avec les premiers moyens d'encliquetage (4.1, 4.2, 4.3) de telle sorte que la plaque coulissante (3) peut occuper une parmi plusieurs positions prédéfinies par rapport à la plaque d'encliquetage (2) et est encliquetée dans la position sélectionnée, le dispositif de logement (9) étant conçu comme unité d'enregistrement d'image (9) et les espacements de moyens d'encliquetage (5.1 à 5.3, 6.1 à 6.3) voisins de la plaque d'encliquetage (2) dans la direction X et dans la direction Y étant inférieurs ou égaux à la zone d'enregistrement d'image de l'unité d'enregistrement d'image (9) et étant supérieurs à la demi-zone d'enregistrement d'image.

2. Dispositif de mesure selon la revendication 1, **caractérisé en ce que** le dispositif de logement (9) est conçu comme caméra de mesure à orifice (9) avec une trajectoire de faisceau d'observation de préférence télécentrée.

3. Dispositif de mesure selon la revendication 2, **caractérisé en ce que** les zones d'image enregistrées dans des positions voisines se chevauchent d'au moins 1/10 de la zone d'enregistrement d'image.

4. Dispositif de mesure selon l'une quelconque des revendications 1 à 3, **caractérisé en ce que** certains des moyens d'encliquetage (4.1, 4.2, 4.3) sont conçus comme des élévations et les autres moyens d'encliquetage (5.1, 5.2, 5.3, 6.1, 6.2, 6.3) sont conçus comme des cavités, les élévations (4.1, 4.2, 4.3) présentant de préférence sensiblement une surface d'appui convexe et les cavités (5.1, 5.2, 5.3, 6.1, 6.2, 6.3) étant conçues comme des rainures, la transition entre deux rainures (5.1, 5.2, 5.3, 6.1, 6.2, 6.3) étant conçue de préférence convexe.

5. Dispositif de mesure selon la revendication 4, **caractérisé en ce que** les seconds moyens d'encliquetage (5.1, 5.2, 5.3, 6.1, 6.2, 6.3) sont répartis en une première zone avec des rainures parallèles et régulièrement espacées et en une seconde zone avec des rainures parallèles et régulièrement espacées, les rainures de la première zone étant sensiblement perpendiculaires aux rainures de la seconde zone.

6. Dispositif de mesure selon l'une quelconque des revendications 1 à 5, **caractérisé en ce que** la plaque d'encliquetage (2) contient au moins un percement (17, 18) pour la fixation d'un objet (19) à mesurer dans la direction X ou dans la direction Y de façon solidaire du coulissement par le biais d'un moyen de fixation, le moyen de fixation (21, 23) étant conçu de telle sorte que l'objet est situé dans la même zone d'enregistrement d'image que l'objet (11) à mesurer de façon coulissante.

7. Dispositif de mesure selon l'une quelconque des revendications 1 à 6, **caractérisé en ce qu'**il est prévu une unité d'analyse (12), qui effectue un calcul d'un ensemble de données en 3D sur la base des données d'enregistrement et **en ce que** également un calibrage du dispositif de mesure (1) est effectué en utilisant un corps de calibrage (25, 26) avec une géométrie connue et les données de calibrage déterminées à partir du calibrage sont utilisées dans l'unité d'analyse (12) lors de l'analyse des enregistrements.

8. Dispositif de mesure selon la revendication 7, **caractérisé en ce que**, lors du calibrage, l'orientation de l'unité d'enregistrement d'image (9) par rapport aux sens de déplacement X, Y de la plaque coulissante (3) et de préférence l'orientation relative dans l'espace de la plaque d'encliquetage (2) par rapport à l'unité d'enregistrement d'image (9) et de préférence la variation de la zone d'enregistrement d'image lors de la modification de la hauteur du dispositif d'enregistrement d'image (9) au-dessus de plaque d'encliquetage (2) sont déterminées.

9. Dispositif de mesure selon l'une quelconque des revendications 1 à 8, **caractérisé en ce que**, sur le côté supérieur de la plaque coulissante (3), il est prévu une possibilité de fixation (15) pouvant pivoter et basculer pour un modèle de dent (11), **en ce que** sur le côté inférieur de la plaque coulissante (3) sont présentes des élévations (4.1, 4.2, 4.3), sur lesquelles la plaque coulissante (3) se trouve et **en ce que** la plaque coulissante (3) peut être sortie du dispositif de mesure et positionnée librement.

10. Dispositif de mesure selon la revendication 9, **caractérisé en ce que** sur le côté supérieur de la plaque coulissante (3) sont placés des repères (16.1, 16.2, 16.3), dont la position correspond à la position des élévations (4.1, 4.2, 4.3) sur le côté inférieur.

11. Dispositif de mesure selon la revendication 9 ou 10, **caractérisé en ce qu'**il est prévu trois élévations (4.1, 4.2, 4.3), dont deux élévations (4.1, 4.3) sont positionnées de telle sorte qu'elles sont parallèles à une arête de la plaque coulissante (3).

12. Procédé pour la mesure en 3D de modèles de dents, un objet (11) à mesurer étant fixé sur une plaque (3) positionnable et l'objet (11) à mesurer étant enregistré par une unité d'enregistrement d'image (9) dans plusieurs prises de vue et un calcul étant effectué par une unité d'analyse (12) pour l'établissement d'un ensemble de données en 3D du modèle de dents (11), **caractérisé par** les étapes suivantes :
- l'élaboration d'une première prise de vue de l'objet (11) à mesurer en une position déterminée par des moyens d'encliquetage (5.1, 5.2, 5.3, 6.1, 6.2, 6.3) d'une plaque d'encliquetage (2) reliée à l'unité d'enregistrement d'image,
- le positionnement de la plaque (3) conçue comme plaque coulissante dans au moins une autre position prédéfinie par des moyens d'encliquetage (5.1 à 5.3, 6.1 à 6.3) voisins de la plaque d'encliquetage (2) et l'élaboration d'une seconde prise de vue par position et
- l'élaboration d'un ensemble de données en 3D par l'analyse des prises de vue élaborées à partir des au moins deux positions différentes prédéfinies de façon précise par des moyens d'encliquetage (5.1 à 5.3, 6.1 à 6.3) voisins, les espacements des positions d'encliquetage de la plaque coulissante (3 ; 25) sur la plaque d'encliquetage (2) étant connus de l'unité d'analyse (12) et l'unité d'analyse (12) assemblant les prises de vue avec un décalage prédéfini par les espacements des positions d'encliquetage de la plaque coulissante (3; 25), sur la plaque d'encliquetage (2).

13. Procédé selon la revendication 12, **caractérisé en ce que** l'unité de prise de vue (9) règle automatiquement la zone nette par des moyens pour le réglage de la zone nette.

14. Procédé selon la revendication 12 ou 13, **caractérisé en ce que** l'unité d'analyse (12) compare les prises de vue individuelles les unes avec les autres dans une zone périphérique des prises de vue pour la localisation relative des prises de vue.

15. Procédé selon l'une quelconque des revendications 12 à 14, **caractérisé en ce qu'**un calibrage du dispositif de mesure (1) est effectué en réceptionnant un corps de calibrage (26) sur une plaque coulissante (25) dans plusieurs positions, et **en ce que** l'unité d'analyse (12) analyse les positions enregistrées et mémorise des données de calibrage.

16. Procédé selon l'une quelconque des revendications 12 à 15, **caractérisé en ce qu'**un déplacement apparaissant lors du réglage en hauteur du dispositif de prise de vue (9), de l'extrait d'image est corrigé automatiquement par l'unité d'analyse (12) du fait que le sens de déplacement constaté lors du calibrage du dispositif de mesure (1) est utilisé pour déplacer des parties d'image identiques de deux images réceptionnées sur des positions voisines le long du sens de déplacement de telle sorte que les deux prises de vue dans la zone périphérique s'harmonisent de la meilleure façon possible.
